Europäisches Patentamt

European Patent Office

Office européen des brevets

(11) Publication number: **0 109 681 B1**

# EUROPEAN PATENT SPECIFICATION

(45) Date of publication of patent specification: **11.03.92**

(51) Int. Cl.⁵: **C07C** 255/37, C07D 403/06, B01J 31/02, //(C07D403/06, 241:00,233:00)

(21) Application number: **83111562.1**

(22) Date of filing: **18.11.83**

(54) process for the preparation of optically-active cyanomethyl esters.

(30) Priority: **22.11.82 US 443513**
**22.11.82 US 443763**
**22.11.82 US 443764**

(43) Date of publication of application:
**30.05.84 Bulletin 84/22**

(45) Publication of the grant of the patent:
**11.03.92 Bulletin 92/11**

(84) Designated Contracting States:
**AT BE CH DE FR GB IT LI LU NL SE**

(56) References cited:
**EP-A- 0 021 925**
**EP-A- 0 063 731**
**EP-A- 0 132 392**
**DE-A- 2 902 466**
**FR-A- 1 363 780**

**Bull. Chem. Soc. Jpn, 59,893-895 (1986)**

(73) Proprietor: **E.I. DU PONT DE NEMOURS AND COMPANY**
**1007 Market Street**
**Wilmington Delaware 19898(US)**

(72) Inventor: **Stoutamire, Donald W.**
**904 Bel Passi Dr.**
**Modesto California 95350(US)**
Inventor: **Tieman, Charles H.**
**2209 Fremont St.**
**Modesto California 95350(US)**
Inventor: **Dong, Walter**
**14628 River Forest**
**Houston Texas 77079(US)**

(74) Representative: **Jones, Alan John**
**CARPMAELS & RANSFORD 43 Bloomsbury Square**
**London, WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF THE CHEMICAL SOCIETY, CHEMICAL COMMUNICATIONS, no. 5, 1981, pages 229-230; JUN-ICHI OKU et al.: "Asymmetric cyanohydrin synthesis catalysed by a synthetic cyclic dipeptide."

CHEMICAL ABSTRACTS, vol. 97, no. 5, August 2, 1982, page 618, ref.nr. 39355x; Columbus, Ohio, US; JUN-ICHI OKU et al.: "Asymmetric cynohydrin synthesis catalyzed by synthetic dipeptides."

CHEMICAL ABSTRACTS, vol. 99, no. 7, August 15, page 524, ref.nr. 53393f; Columbus, Ohio, US; &JP-A-83 29 757

LIEBIGS: ANNALEN DER CHEMIE, vol. 634, 1960, pages 9-22; H. PRACEJUS: "Organische Katalysatoren, LXI 1): Asymmetrische Synthesen mit Ketenen, I, Alkaloid-katalysierte asymmetrische Synthesen fon alpha-Phenyl-propionsäureestern."

LIEBIGS: ANNALEN DER CHEMIE, vol. 722, 1969, pages 1-11; H. PRACEJUS et al.: "Asymmetrische Synthesen mit Ketenen, VII 1): Tertiäre Amine mit einem asymmetrischen C-Atom als Katalysatoren für die asymmetrische Synthese von alpha-Phenyl-propionsäure-methyl-ester."

BULLETIN OF THE CHEMICAL SOCIETY OF JAPAN, vol. 50, no. 6, June 1977, pages 1532-1534; T. YAMASHITA et al.: "Asymmetric reactions. II. Asymmetric of methyl alpha-phenylpropionate by means of chiral polymers."

CHEMICAL ABSTRACTS, vol. 85, 1976, pages 214/5, ref.nr. 173630p, and the Ninth Collective Index, vol. 76-85, 1972-1976, page 18751 CS; Columbus, Ohio, US; P. FELKER: "A gas-liquid chromatopgraphic-isotope dilution analysis of cysteine, histidine, and tryptophan in acid-hydrolyzed protein."

## Description

The present invention is directed to a process for the preparation of optionally substituted S-alpha-cyano-3-phenoxybenzyl alcohol intermediates.

Stereoisomers of chiral-cyanomethyl esters of alpha-chiral carboxylic acids or the acids themselves usually have different effects in biological systems. In the past, it usually had not been simple to prepare such optically-active cyanomethyl esters directly because the corresponding chiral alpha-hydroxynitriles were not always readily available or synthesis methods gave products with low or no enrichment. Even when these optically-active alpha-hydroxynitriles were available or became more readily available, the optically-active acids were not always readily accessible. Often, the optically-active acids were obtained by classical resolution, which was usually time consuming and not practical on a large scale. Optically-active alpha-hydroxybenzeneacetonitriles are known in the art and are of interest, per se, and as intermediates, e.g. to esters. In alcohol derived pyrethroid esters, those having an (alpha-S)-alpha-hydroxynitrile moiety coupled with the appropriate pyrethroid acid usually have the highest pesticidal activity. However, such (alpha-S)-alpha-hydroxynitriles have not been easily obtained in the past because they were usually prepared by resolution.

Journal of the Chemical Society, Chemical Communications (1981), pp 229 and 230 discloses the asymmetric addition of hydrogen cyanide to benzaldehyde catalysed by cyclo(L-phenylalanyl-L-histidine) to give a very high yield of the corresponding cyanohydrin [HO-CH(Ph)-CN].

DE-A-2902466 discloses (S)-alpha-cyano-3-phenoxybenzyl alcohol and its preparation involving as an essential feature the use as an intermediate product of the optically active ether, (1R, 5S)-6,6-dimethyl-4(R)-[(S)-cyano-(3'-phenoxyphenyl)-methoxy]-3 oxabicyclo [3.1.0] hexan-2-one.

According to this invention an optically-active, optionally substituted-S-alpha-cyano-3-phenoxybenzyl alcohol or a mixture enriched therein is prepared by treating an optionally substituted 3-phenoxybenzaldehyde with a source of hydrogen cyanide in the presence of a substantially water-immiscible, aprotic solvent and in the presence of a cyclo(D-phenylalanyl-D-histidine) dipeptide catalyst.

The catalyst is an optically-active histidine-containing cyclic peptide. The catalyst is prepared by conventional peptide synthesis, for example, as in Greenstein, J.P. and M. Winitz, "Chemistry of the Amino Acids", John Wiley & Sons, Inc., New York, 1961.

The asymmetric carbons of the catalyst have the D-configuration.

A substantially water-immiscible, aprotic solvent for use in this invention is defined as an aprotic solvent in which the solubility in water is not more than 5%v. For example, the solvent is a hydrocarbon, or ether solvent including acyclic, alicyclic, alicyclic or aromatic materials. Preferably, at the reaction temperature the solvent has a boiling point below about 150°C (and does not interfere with the reaction). For example, suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between 40° and 65°C, between 60° and 80° or between 80° and 110°C. Petroleum ether is also suitable. Cyclohexane and methylcyclohexanes are examples of useful cyclohexanes containing from 6 to 8 carbon atoms. Aromatic hydrocarbon solvents can contain from 6 to 10 carbon atoms, for example, benzene, toluene, o-, m- and p-xylene, the trimethylbenzenes and p-ethyltoluene. Useful ethers include diethyl ether, diisopropyl ether and methyl-t-butyl ether. Preferably, the solvent is an aromatic hydrocarbon, especially toluene, diisopropyl ether or diethyl ether or mixtures thereof (e.g. 25/75 of diethyl ether/toluene).

The reaction to prepare the optically-active S-alpha-cyano-3-phenoxybenzyl alcohols is suitably conducted by adding the aldehyde and solvent to the cyclo(D-phenylalanyl-D-histidine) catalyst, dispersing (mechanical grinding or agitating the mixture, e.g. by stirring), adding hydrogen cyanide and maintaining the reaction conditions for an amount of time to effect the formation of the optically-active alpha-hydroxynitrile. That is, preferably, the hydrogen cyanide is introduced concurrently with, subsequent to or rapidly followed by the solvent and/or aldehyde to increase the conversion and stereoselectivity. The presence of cyanide ions has an adverse effect on the catalyst in this reaction and competing racemization is reduced by protecting the catalyst from cyanide ions. The forming and maintaining of a well dispersed but not necessarily homogeneous-like reaction mixture are useful. Separation and recovery of the optically-active alcohol product are achieved by conventional techniques, including extraction and the like.

The temperature of the reaction as well as the pressure can vary. At normal pressures, the temperature is from about -10°C to about 80°C. Preferably, ambient temperatures of about 5°C to about 35°C are convenient to give good yield, rate of reaction and enantiomeric excess of the desired optically-active product, the lower temperature of 5°C giving good results.

Preferably, from 1 to 3 moles of hydrogen cyanide are used per mole of the optionally substituted 3-phenoxybenzaldehyde. The amount of catalyst used in making the optically-active S-alpha-hydroxynitrile, can vary. For example, it can be used in the range of from about 0.1 to about 10 mole percent and, preferably 0.1 to about 5 mole percent based upon the weight of the aldehyde present, especially about 1.5 to about 7.5 mole percent. The catalyst is preferably well dispersed in the reaction mixture.

When the catalyst is prepared by conventional methods in the presence of solvent (e.g. water), it can also contain solvent (e.g. water) of crystallization. The optically-active cyclo(D-phenylalanyl-D-histidine) dipeptide catalyst of the invention thus includes the presence or absence of solvent (e.g. water) of crystallization.

Preparation of the catalyst

1 - N-(Benzyloxycarbonyl)-D-phenylalanine

A 15.0 g sample of D-phenylalanine was dissolved in 45 ml of aqueous solution containing 7.26 g of 50% sodium hydroxide. This solution was stirred at 0-10°C as 16.3 g of benzyl chloroformate was added rapidly in portions. The resulting reaction was mildly exothermic, and shortly after addition, solids precipitated. An additional 45 ml of water and 3.63 g of 50% sodium hydroxide were added, causing most of the solids to redissolve. The reaction mixture was stirred for 20 minutes and then acidified with 6 N hydrochloric acid. The resulting solids were filtered, washed with water and then with hexane, and dried by suction and then under vacuum to give 47 g of white solids. These solids dissolved in ether were washed twice with 1 N hydrochloric acid and then with water, dried over MgSO$_4$ and stripped to 35°C at 2.5 mm Hg to give 27.7 g of the desired product as a colorless oil.

2 - N-(Benzyloxycarbonyl)-D-phenylalanine, p-nitrophenyl Ester

A 300 ml three-neck flask with stirrer and dropping funnel was charged under a nitrogen atmosphere with 27 g of the acid of Embodiment 1 above in 135 ml of pyridine, followed by 13.2 g of p-nitrophenol. The resulting solution was cooled to 0° to 10°C as 14.6 g of phosphorus oxychloride was added. The resulting mixture was warmed to 25°C, stirred for 15 minutes, then poured into 300 ml of ice water. Filtration of the resulting solid, followed by washing with water and drying by suction, gave 33 g of product. This was crystallized from 340 ml of hot ethyl alcohol with chilling to -5°C. The product was filtered, washed with chilled ethyl alcohol, then with hexane, and sucked dry to give 28.7 g of the desired product, m.p. 122.5-124.5°C, $[\alpha]_D^{23}$ +24.7 (c 2.0, dimethylformamide).

3 - N-(Benzyloxycarbonyl)-D-phenylalanyl-D-histidine Methyl Ester

To a stirred solution of 5.0 g of D-histidine methyl ester hydrochloride in 40 ml of methylene chloride was added 4.18 g of triethylamine followed by 8.27 g of the nitrophenyl ester prepared as in Embodiment 2 above. The reaction mixture immediately became bright yellow and solids began to precipitate. The reaction mixture was stirred for 2 hours, then stored overnight at -10°C. The reaction mixture was rewarmed to room temperature, and 0.6 ml of triethylamine was added. Then, 490 mg of the D-histidine methyl ester hydrochloride was added, and stirring was continued for 2 hours. The reaction mixture was washed with 20 ml of water, then twice with 20 ml of 10% ammonium hydroxide, and then twice with 20 ml of water. All the washes were back-extracted serially with 20 ml of methylene chloride, and the combined organic phases was dried with MgSO$_4$ and stripped to 100 ml, filtered through silica, followed by 25 ml of 20% methanol in ethyl acetate. The resulting eluate was stripped to 40 ml and diluted to 120 ml with diethyl ether; the precipitated solid was filtered, washed with diethyl ether, and dried by suction to give 5.66 g of the desired product as a white solid, m.p. 114.5-117°C $[\alpha]_D^{20}$ -55.5 (c 2 in CHCl$_3$).

4 - Cyclo(D-phenylalanyl-D-histidine)

5.60 g of methyl ester of Embodiment 3 above was stirred and hydrogenated in 100 ml of methanol over 220 mg of 10% palladium on carbon at atmospheric pressure. After 3 hours, solids began to precipitate; an additional 2.5 ml of methanol was added to facilitate stirring. After 7 hours, an additional 280 ml of methanol was added as the mixture was heated to reflux. The mixture was filtered hot, and the filtrate was stripped to a gel-mush, which was mixed with 100 ml of diethyl ether. The resulting solid was filtered, washed with diethyl ether, and dried by suction and then under high vacuum at 35°C to give 3.29 g of the desired product as an off-white powder, $[\alpha]_D^{23}$ = +68.5 (c 2.0 in CH$_3$COOH).

4

Example 1 - S-alpha-Cyano-3-phenoxybenzyl Alcohol

A 100 ml three-neck Bantam-ware flask was charged with 43 mg of cyclo(D-phenylalanyl-D-histidine) and put under a nitrogen atmosphere. Then, 3.51 ml of hydrogen cyanide was added by syringe causing the catalyst to swell and become a gel. After 5 minutes, 30 ml of toluene was added, causing additional catalyst to precipitate. 5.95 g of 3-phenoxybenzaldehyde was added all at once. The reaction mixture was stirred for 4.75 hours and then quenched with 20 ml of water containing 10 drops of concentrated hydrochloric acid. The toluene solution was separated, washed twice with water, and diluted to 50 ml with toluene for analysis, which showed 80% S-alpha-cyano-3-phenoxybenzyl alcohol isomer was produced.

Example 2 - S-alpha-Cyano-3-phenoxybenzyl Alcohol

The reaction of Embodiment 5 above was repeated using 171 mg of cyclo(D-phenylalanyl-D-histidine). At intervals, 0.25 ml samples were removed and examined by gas liquid chromatography as follows:

| Time | % Conversion of Aldehyde |
|---|---|
| 35 minutes | 20 |
| 2 hours | 76 |
| 6.5 hours | 95 |

After 7 hours, the reaction mixture was quenched by addition of 10 ml of 1 N hydrochloric acid. The organic phase was separated and washed twice with water, dried over $MgSO_4$, filtered and stored at -10°C. The filtrate was diluted to 50 ml with toluene and the optical rotation was determined to be -1.54° at 21° in 1 dm cell. A sample of the product was acetylated with p-nitrophenylacetic anhydride and the stereoisomer ratio was determined by HPLC on a chiral Pirkle column to be 71% S-alpha-cyano-3-phenoxybenzyl alcohol and 29% R-alpha-cyano-3-phenoxybenzyl alcohol.

Example 3 - S-alpha-Cyano-3-phenoxybenzyl Alcohol

Two small round-bottom flasks having magnetic stirrers and septum covers were each charged with 22.5 mg of cyclo(D-phenylalanyl-D-histidine) and put under nitrogen. A sample of 0.98 ml of hydrogen cyanide was diluted to 25 ml with toluene, and 5 ml of the solution was added via syringe to each flask. After about 5 minutes, 0.87 ml of 3-phenoxybenzaldehyde (POAL) was added to each flask. Flask No. 1 was stirred in an oil bath at 35°C and flask No. 2 was stirred in a water bath at 24-26°C. The results of these experiments are below.

| Time, hr | Flask No. 1 | | | Flask No. 2 | | |
|---|---|---|---|---|---|---|
| | POAL, % | α-Hydroxynitrile, % | R/S | POAL, % | α-Hydroxynitrile, % | R/S |
| 0.5 | 19 | 81 | 8.7/91.3 | 18 | 82 | 15.8/84.2 |
| 1 | 12 | 88 | - | 13 | 87 | 14.4/85.6 |
| 2 | 12 | 88 | - | 12 | 88 | - |
| 4 | 10 | 90 | 11.4/88.6 | 12 | 88 | 19 0/81.0 |
| 8 | 10 | 90 | - | 13 | 87 | - |

Example 4 S-alpha-Cyano-3-phenoxybenzyl Alcohol

A reaction was conducted by contacting 0.0099 m/kg cyclo(D-phenylalanyl-D-histidine) with 0.99 m/kg of 3 phenoxybenzaldehyde followed by 2 2 m/kg of hydrogen cyanide and 190 ppm water. The reaction was conducted in toluene at 25°C. The product obtained with 93% conversion of aldehyde was 88% S-alpha-cyano-3-phenoxybenzyl alcohol isomer.

Example 5 S-alpha-Cyano-3-phenoxybenzyl Alcohol

To 0.2933 g of cyclo(D-phenylalanyl-D-histidine) in 15 ml of diethyl ether at 25°C was added 2.907 g of 3-phenoxybenzaldehyde followed by 0.940 g of hydrogen cyanide. After 2 hours, 94% of the 3-phenoxybenzaldehyde had been converted and the product had an S-alpha-cyano-3-phenoxybenzyl alcohol enantiomeric excess of 84%. After 4 to 20 hours, 99.4% of the 3 phenoxybenzaldehyde had been converted and the product had an S-alpha-cyano-3-phenoxybenzyl alcohol enantiomeric excess of 67-68%.

Example 6 S-alpha-Cyano-3-phenoxybenzyl Alcohol

A reaction was conducted by treating 0.0200 g of cyclo(D-pnenylalanyl-D-histidine) at 25°C under nitrogen with 1.4037 g of 3-phenoxybenzaldehyde to disperse the catalyst followed by injecting 2.1812 g of toluene with 13.65 w% hydrogen cyanide. After 2.7 hours, 93% of the 3-phenoxybenzaldehyde was converted and the product had an S-alpha-cyano-3-phenoxybenzyl alcohol enantiomeric excess of 77%.

Example 7 - S-alpha-Cyano-3-phenoxybenzyl Alcohol

A reaction was conducted at 25°C by contacting 0.0519 g of cyclo(D-phenylalanyl-D-histidine) in 9.32 ml of diethyl ether with 1.811 g of 3-phenoxybenzaldehyde followed by 0.617 g of hydrogen cyanide. After 3.6 hours, 99.4% of the 3-phenoxybenzaldehyde had been converted and the product had an S-alpha-cyano-3-phenoxybenzyl alcohol enantiomeric excess of 72%.

**Claims**

1. A process for the preparation of an optionally substituted S-alpha-cyano-3-phenoxybenzyl alcohol or a mixture enriched therein which comprises treating an optionally substituted 3-phenoxybenzaldehyde with a source of hydrogen cyanide in the presence of a substantially water-immiscible aprotic solvent and a cyclo(D-phenylalanyl-D-histidine) dipeptide catalyst.

2. A process according to claim 1 wherein the solvent is an aromatic hydrocarbon or an ether or a mixture thereof.

3. A process according to claim 2 wherein the solvent is toluene or diethyl ether or a mixture thereof.

4. A process according to claim 1 wherein from 1 to 3 moles of hydrogen cyanide is used per mole of the optionally substituted 3-phenoxybenzaldehyde.

5. A process according to claim 1 wherein the hydrogen cyanide is introduced concurrently with, subsequent to or rapidly followed by the solvent and/or aldehyde.

6. A process according to claim 1 wherein the aldehyde is 3-phenoxybenzaldehyde.

**Revendications**

1. Un procédé pour la préparation d'un alcool S-alpha-cyano-3-phénoxybenzylique facultativement substitué ou d'un mélange enrichi en celui-ci, qui consiste à traiter un 3-phénoxybenzaldéhyde facultativement substitué par une source de cyanure d'hydrogène en présence d'un solvant aprotique sensiblement non miscible à l'eau et d'un catalyseur dipeptidique qui est la cyclo(D-phénylalanyl-D-histidine).

2. Un procédé selon la revendication 1, dans lequel le solvant est un hydrocarbure aromatique ou un éther ou un mélange d'entre eux.

3. Un procédé selon la revendication 2, dans lequel le solvant est le toluène ou l'éther de diéthyle ou un mélange d'entre eux.

4. Un procédé selon la revendication 1, dans lequel on utilise 1 à 3 moles de cyanure d'hydrogène par mole du 3-phénoxybenzaldéhyde facultativement substitué.

5. Un procédé selon la revendication 1, dans lequel le cyanure d'hydrogène est introduit en même temps que, après, ou peu de temps avant le solvant et/ou l'aldéhyde.

**6.** Un procédé selon la revendication 1, dans lequel l'aldéhyde est le 3-phénoxybenzaldéhyde.

**Patentansprüche**

**1.** Verfahren zur Herstellung eines gegebenenfalls substituierten S-Alpha-cyano-3-phenoxybenzylalkohols oder eines damit angereicherten Gemisches, bei dem ein gegebenenfalls substituierter 3-Phenoxybenzaldehyd mit einer Quelle von Cyanwasserstoff in Gegenwart eines im wesentlichen wasser-nichtmischbaren aprotischen Lösungsmittels und einem Cyclo(D-phenylalanyl-D-histidin)dipeptid-Katalysator behandelt wird.

**2.** Verfahren nach Anspruch 1, bei dem das Lösungsmittel ein aromatischer Kohlenwasserstoff oder ein Ether oder ein Gemisch derselben ist.

**3.** Verfahren nach Anspruch 2, bei dem das Lösungsmittel Toluol oder Diethylether oder ein Gemisch derselben ist.

**4.** Verfahren nach Anspruch 1, bei dem 1 bis 3 Mol Cyanwasserstoff je Mol des gegebenenfalls substituierten 3-Phenoxybenzaldehyds verwendet wird.

**5.** Verfahren nach Anspruch 1, bei dem der Cyanwasserstoff gleichzeitig mit dem Lösungsmittel und/oder Aldehyd, anschliessend daran oder schnell gefolgt von Lösungsmittel und/oder Aldehyd eingeführt wird.

**6.** Verfahren nach Anspruch 1, bei dem der Aldehyd 3-Phenoxybenzaldehyd ist.